Europäisches Patentamt

European Patent Office

Office européen des brevets

(11) Publication number: **0 179 584 B1**

# EUROPEAN PATENT SPECIFICATION

(45) Date of publication of patent specification: **18.03.92**   (51) Int. Cl.⁵: **B01J  23/50**, B01J 35/02,
C07D 301/10, C07D 303/04

(21) Application number: **85307070.4**

(22) Date of filing: **02.10.85**

(54) **Production of alkylene oxides and catalysts therefor.**

(30) Priority: **25.10.84 GB 8426996**

(43) Date of publication of application:
**30.04.86 Bulletin  86/18**

(45) Publication of the grant of the patent:
**18.03.92 Bulletin  92/12**

(84) Designated Contracting States:
**BE DE FR GB IT NL SE**

(56) References cited:
**EP-A- 0 001 078        EP-A- 0 003 642
DE-A- 1 920 976        FR-A- 1 137 622
FR-A- 2 365 625        GB-A- 2 057 905
US-A- 2 408 164        US-A- 2 805 229
US-A- 4 471 071**

(73) Proprietor: **IMPERIAL CHEMICAL INDUSTRIES
PLC
Imperial Chemical House, Millbank
London SW1P 3JF(GB)**

(72) Inventor: **Langley, Philip Edward
Temple House
Carlton in Cleveland Cleveland(GB)**
Inventor: **Kirk, Frank Alfred
Crawford House Long Street
Easingwold York(GB)**

(74) Representative: **Roberts, Jonathan Winstanley
et al
Imperial Chemical Industries PLC Legal De-
partment;Patents Bessemer Road PO Box 6
Welwyn Garden City Hertfordshire AL7
1HD(GB)**

EP 0 179 584 B1

## Description

This Invention relates to the production of alkylene oxides and catalysts therefor.

It is known to produce alkylene oxides, especially ethylene oxide, by contacting gases comprising the corresponding olefine and oxygen with catalyst comprising silver on a support which may be an -alumina support. Such catalysts may be in the form of hollow cylindrical pellets.

A method of forming catalysts, for example Friedel-Crafts metal halide catalysts, into pellets of a wide variety of shapes by heating the solid catalytic material to a temperature below its melting point and applying pressure to fuse particles thereof at their points of contact is described in US patent 2,408,164. Among many shapes which can be formed by that process are multiholed cylinders.

German OLS 1,920,976 teaches that a wide variety of shapes of catalyst pellets may be used in ethylene oxide catalysts. Cylindrical, spherical, spheroidal and saddle and ring shapes were mentioned.

The use of ring shaped ethylene oxide catalyst pellets is disclosed in US patents 3,962,136 and 4,471,071. These can be regarded as hollow cylindrical pellets. Similar teaching on pellet shape appears to be contained in US patent 4,458,032.

We have found that the performance of such catalysts may be improved by modifying the shape of the pellets.

This invention therefore comprises a catalyst for the production of an alkylene oxide, (especially ethylene oxide), by contacting the corresponding olefine and oxygen with the catalyst, which comprise silver deposit on a porous heat resisting support, in which the catalyst is in the form of substantially cylindrical pellets in which the ratio of the length to the diameter is in the range 2:1 to 1:2 pierced by 3 to 15 substantially longitudinal holes passing completely through the pellets, the total volume of the holes within the pellet being 5 to 25% of the volume of the pellets including the holes, the disposition of the holes across the cross section of the pellet facilitating access of gas to the substance of the pellet.

By "substantially cylindrical" we include pellets of which the outer boundary of the transverse cross section comprises flat surfaces, provided that the angles between such surfaces are not less than about 120°. The pellets may thus be for example hexagonal, or octagonal, but it is preferred that the outer boundary should be substantially circular. As the pellets will normally be made by extruding, cutting, for example by blades, wires or saws and calcining there may be a tendency for some acceptable distortion to occur due to gravity or heating effects before the shape is finally set.

The end faces of the pellets may be at right angles to the axis or if desired, may be at an angle to this, for example if it is wished to alter the packing characteristics of the pellets.

The holes may be of any cross section; for example they may be sector shaped and arranged so that in cross section the pellet resembles a wheel with a rim and spokes, but they are suitably round. The holes are preferably arranged so as to minimise the maximum diffusion path of gases from the surfaces to the interior parts of the substance of the pellets.

The catalyst suitably comprises silver supported on a porous heat resisting support which has a specific surface area in the range 0.05 to 10 $m^2/g$ and preferably 0.1 to 5 $m^2/g$ and more preferably 0.3 to 3 $m^2/g$ as measured by the Brunauer, Emmett and Teller method. The catalyst may be produced as disclosed in European patent No. 3642.

The catalyst support preferably has an apparent porosity as measured by the water absorption method of at least 20%, for example 25-70% preferably 30-65% and mean pore diameters of 0.1 to 20 microns preferably 0.3 to 4 microns as measured by the mercury porosimetry method. The pore size distribution of the support may be bi-or tri-modal, in which case the pores of diameters up to 2 microns preferably account for 30 to 70% of the total pore volume the pores of diameter 2 to 20 microns preferably 0 to 50% of the total pore volume, and the larger pores preferably 20 to 50% of the total pore volume.

Most of the silver content of the catalyst is preferably present in the form of discrete particles adhering to the support having equivalent diameters of less than 10,000Å preferably in the range 20-10,000Å and more preferably 40-8,000Å for example 100-5,000Å. By equivalent diameter is meant the diameter of a sphere of the same silver content as the particle.

Preferably at least 80% of the silver is present as particles having equivalent diameters in the aforesaid range, the quantity of silver being judged in terms of the number of particles falling in that range. The silver is believed to be present largely as metallic silver. The dimensions of the silver particles may be determined by scanning electron microscopy.

The support may be an alumina, silicon carbide, silica, zirconia, titania or silica/alumina support, but it is preferably composed of an aggregate of alpha-alumina particles which may be fused together or cemented together with, for example silica titania or baryta.

The catalyst preferably comprises 3 to 50% and more preferably 3 to 30% for example 6 to 28% by weight of silver.

It is preferred that the catalyst should contain cations, for example alkali and/or alkaline earth metal cations as the corresponding nitrate or nitrite or in a form capable of reacting to produce the corresponding nitrate or nitrite by reaction with nitrate and/or nitrite forming substances in accordance with our European Patent 3642. This is especially preferred if the catalyst is treated with the nitrate or nitrite forming substance intermittently in order to restore its selectivity. It is believed that an effect of such cations is to hold more nitrate and/or nitrite ions on the catalyst when it is in use or to hold them longer than is the case in their absence but we do not wish to be bound by any theory as to their action.

The cations may be introduced to the support before during or after the introduction of the silver compound. Preferably the cations are introduced to a composition in which the silver is present in metallic form on a support. The cations are suitably introduced as solutions in water and/or organic solvents. If it is desired to impregnate a catalyst which has already been used in the oxidation of an alkene to an alkylene oxide and has lost performance, this may be carried out also. Suitable concentrations of such cations in a form which is extractable by water may be for example 50 to 5000 parts per million of alkali metals by weight and/or 50 to 20,000 parts per million of alkaline earth metals by weight.

The cations may be provided for example as nitrates, hydroxides, carbonates, bicarbonates or carboxylates, for example lactates or more preferably formates or oxalates.

The catalyst preferably contains rubidium, cesium, lithium, cadmium, calcium, magnesium, strontium and/or barium and/or more preferably sodium and/or potassium, these elements being present in a form which permits the extraction of their ions from the catalyst by water.

Partial pressures of ethylene or propylene in processes according to the invention may be in the ranges 0.1 - 30 and preferably 1 to 30 bars. The total pressure may be in the range of from 1 to 100 and preferably 3 - 100 bars absolute. The molar ratio of oxygen to ethylene or propylene may be in the range 0.05 to 100. The partial pressure of oxygen may be in the range 0.01 and preferably 0.1 to 20 bars and preferably 1-10 bars. The oxygen may be supplied for example in the form of air or preferably as commercial oxygen. A diluent for example helium, nitrogen, argon, carbon dioxide and/or methane may be present in proportions of 10-80% and preferably 40-70% by volume in total. Suitably the diluent comprises methane as aforesaid together with, for example 100 to 20,000 parts per million by volume of ethane, preferably together with small amounts, for example 10 to 10,000 parts per million by volume of $C_3$ to $C_6$ alkanes, cycloalkanes or alkenes preferably propylene, cyclopropane, isobutene or isobutane. It is necessary to operate using gas compositions which are outside the explosive limits.

The temperature is suitably in the range 180 to 320°C, preferably 200 to 300°C and more preferably in the range 220 to 290°C. Contact times should be sufficient to convert 0.5 - 70%, for example 2 to 20 and preferably 5 - 20% of the ethylene and unconverted ethylene is, after separation of the product, suitably recycled, optionally in the presence of unconverted oxygen where appropriate and suitably after partial removal of $CO_2$. Suitably 15 to 50% and preferably 25 to 40% of the oxygen fed is consumed. It is preferred that the $CO_2$ content should be in the range 1% to 8% and more preferably 1.5% to 6% by volume.

The chlorine-containing reaction modifier may be of known type. It is preferred that it should be a $C_1$ to $C_{10}$ compound also containing hydrogen. It may be for example 1,1 or preferably 1,2-dichloroethane but it is preferred to use methyl chloride or more preferably vinyl chloride. Chlorinated aromatic compounds, for example chlorobenzene, dichlorobenzene and chlorinated toluenes are also suitable. It is preferred that the concentration of the chlorine containing reaction modifier should be in the range 0.1 -500 and preferably 1 to 50 parts per million parts of the reaction medium by volume.

If a nitrate or nitrite forming substance is used it may be supplied continuously to the process of producing an olefine oxide at a low level for example 0.1 and preferably 0.2 to 200 and preferably 0.5 to 50 parts per million of $NO_2$ equivalent of the process gas by volume. Not all substances have the same efficiency in forming nitrate or nitrite ions in the catalyst. By $NO_2$ equivalent is meant the amount of the substance which is equivalent to the specified amounts of $NO_2$. In general one mole of NO, 0.5 moles of $N_2O_4$, and under typical conditions about ten moles of ammonia, three moles of ethylene diamine and two moles of acetonitrile are equivalent to one mole of $NO_2$. The values must however be determined experimentally for each compound under the appropriate conditions either by determining the amount equivalent to the desired amount of $NO_2$ under operating conditions or (less suitably) by determining the quantity of $NO_3$ and $NO_2$ ions deposited on a catalyst bed under standard conditions compared with the quantity deposited by $NO_2$.

## EXAMPLE

High purity alpha alumina powder milled and sieved to less than 45 micron particle size, with a crystal size of about 3 micron, was mixed with plasticiser and a water insoluble pore forming agent in a tumbling mixer and water was added gradually to the combined powder, whilst slowly stirring in an open bowl. To ensure thorough distribution of the water the damp powder was pugged twice by screw feeding through a multi hole die plate thus forming a very stiff paste.

The stiff alumina paste was divided into two equal portions. The first portion was extruded using a British Ceramic Research Association two inch screw extruder equipped with a single hole die plate containing a seven pin core rod. The extrudate rope emerging from the die was taken and cut into uniform pellet lengths whilst still wet using a guillotine.

The wet pellets were dried in an oven set at 100°C to give hard, dry seven hole pellets. Single hole pellets were also produced, in exactly the same way as before, the seven pin core rod in the extruder die being replaced by a single core rod in this case and using the other portion of the alumina paste.

Firing was carried out in an electric kiln at 1450°C. Both samples were fired at the same time, and in the process the plasticiser and pore forming agent were totally burned away to leave batches of lightly sintered porous alpha alumina support pellets containing either seven holes or a single hole. These catalyst support pellets comprising high purity -alumina containing $450 \pm 150$ ppm silica expressed as silicon and less than 100 ppm alkali metal in the form of cylinders 8mm diameter and 8mm long pierced in one case by a single central longitudinal hole 3mm in diameter and in the other case by seven longitudinal holes 1.22 mm in diameter one being central and the others being regularly spaced on a circle of 4.39mm diameter centred on the axis of the pellet (this giving a similar total volume of the holes) were converted to catalyst as described below. The mean pore diameter was 2.4 microns the water porosity was $0.31 \pm 0.02$ ml/g and the surface area was 0.52 m$^2$/g.

Two catalysts were prepared according to the same procedure described below.

Silver nitrate crystals (8100 g) were dissolved in distilled water (1644 ml) to form a concentrated solution. Mono isopropylamine (5838g) was added slowly to this solution whilst stirring and cooling, to give a clear solution of silver nitrate/mono isopropylamine complex containing 3.8% w/w excess mono isopropylamine. This stock of silver/amine complex solution was used to make both batches of catalyst. 13.075kg of seven hole support and 12.105 kg of single hole support were used in making two batches of catalyst.

Each support was immersed in the solution at ambient temperatures for a period of 20 minutes before being removed and allowed to drain. The support, wet with silver nitrate/amine complex solution, was charged to a perforated basket which was then loaded into a catalyst preparation unit. The impregnated support was heated in a stream of circulating nitrogen, the temperature being gradually increased from 100°C to 240°C over a period of 18 hours. The silver nitrate/amine complex decomposed reductively to leave finely dispersed silver on the alumina. The product was then washed with boiling water for 16 hours, dried and impregnated with a solution of potassium formate (89.3 g) in a water (115 ml) and methanol (22.7751) mixture. This solution was divided between the two batches of catalyst. In each case, the washed, silvered support was totally immersed in circulating potassium formate/methanol solution for 20 minutes. This solution was removed and the silvered support allowed to drain for 5 minutes. The vessel holding the silvered support wet with potassium formate/methanol was then closed and allowed to stand for 16 hours. Each batch of catalyst was dried in a stream of hot nitrogen.

The catalysts were tested as follows. Reactor tubes of 39 mm internal diameter were charged with each catalyst to give bed lengths of 10.7 m (bed volume 12.8 1.) and 3 m of tube length above each catalyst was packed with inert alumina pellets. The tubes were each surrounded by a liquid heat exchange fluid for temperature control. At startup, a reactor feed gas composition of $0_2$ 5%, $C_2H_4$ 27%, vinyl chloride 6 ppm, ethane 0.3%, NO plus $NO_2$ 10 ppm was established the balance being substantially methane with a small proportion of inert gases. The feed to each reactor was at 75⁻C 18.5 bar pressure, with a gas hourly space velocity of 3200 hr⁻¹. Reactor temperatures were then raised to initiate reaction. Oxygen, ethylene, ethane, methane, NO and vinyl chloride feeds were then mixed with recycle gas to give the desired feed composition. After fifteen days on line 1.22 m$^3$/hr oxygen, 1.42 m$^3$/hr ethylene, 15 l./hr ethane, 730 ml/hr nitrous oxide in methane and 36 ml/hr vinyl chloride in methane all expressed as vapour at standard temperature and pressure plus an intermittent flow of methane sufficient to control the plant pressure were mixed with recycle gas to give a reactor feed composition as follows:

$O_2$ 7.4%, $C_2H_4$ 27%, $CO_2$ 1.6%, vinyl chloride 7.8 ppm, ethyl chloride 4.1 ppm, ethane 0.3%, $NO_2$ 7.8 ppm, NO 4.1 ppm, the balance being substantially methane. The reaction feed was at 75⁻C, 18.5 bar with a gas hourly space velocity of 3200 hr⁻¹ and an oxygen conversion across each reactor of 20%. The seven hole pellets gave a selectivity of ethylene converted of 86.1°% at 257°C average catalyst temperature and the single hole pellets gave a selectivity of 85.3% at 258°C average catalyst temperature. Thus seven hole pellets were both more selective and more active. No difference in pressure drop to maintain the flow rates through the reactor tubes was noticed. The product gases from each reactor were cooled to 75°C before being combined and contacted with 250 l./hr of water at 20°C, to absorb product ethylene oxide. The resulting gas stream was then split, part being contacted with 250 l./hr of 0.5% w/w caustic soda solution in a carbon dioxide scrubber. The scrubber gaseous product and the bypass stream were combined and then compressed to form the recycle gas stream.

ppm means, in the case of liquids or solids, parts per million by weight and in the case of gases, parts per million by volume. Gas hourly space velocities are calculated as at standard temperature and pressure.

## Claims

1. A catalyst for the production of an alkylene oxide, by contacting the corresponding olefine and oxygen with the catalyst, which comprises silver deposited on a porous heat resisting support, in which the catalyst is in the form of substantially cylindrical pellets in which the ratio of the length to the diameter is in the range 2:1 to 1:2, pierced by 3 to 15 substantially longitudinal holes passing completely through the pellets, the total volume of the holes within the pellet being 5 to 25% of the volume of the pellets including the holes, the disposition of the holes across the cross section of the pellet facilitating access of gas to the substance of the pellet.

2. A catalyst as claimed in claim 1 in which the support is composed of an aggregate of alpha alumina particles which are fused or cemented together.

3. A catalyst as claimed in any preceding claim which has a specific surface area in the range 0.05 to 10 $m^2/g$ and a water absorption of at least 20% which comprises 3 to 30% by weight of silver and in which at least 80% of the silver is present as particles having equivalent diameters of less than 10,000Å.

4. A catalyst as claimed in any preceding claim which comprises alkali and/or alkaline earth metal cations as the corresponding nitrate and/or nitrite or in a form capable of reacting to produce the corresponding nitrate or nitrite by reaction with nitrate and/or nitrite forming substances in a gas phase.

5. A process of producing an alkylene oxide which comprises contacting a gas phase comprising an olefine, oxygen, a chlorine containing reaction modifier and a nitrate and/or nitrite forming substance with a catalyst as claimed in any preceding claim.

6. A process as claimed in claim 5 in which the catalyst comprises an alkali metal in a form which permits its extraction from the catalyst by water.

7. A process as claimed in claim 5 or 6 in which the alkylene oxide is ethylene oxide and the olefine is ethylene.

## Revendications

1. Catalyseur pour la production d'un oxyde d'alkylène par mise en contact de l'oléfine correspondante et d'oxygène avec le catalyseur, lequel comprend de l'argent déposé sur un support poreux résistant à la chaleur, caractérisé en ce que le catalyseur est sous la forme de pelletes pratiquement cylindriques dans lesquelles le rapport de la longueur au diamètre est de l'ordre de 2:1 à 1:2, traversé complètement par 5 à 15 trous pratiquement longitudinaux, le volume total des trous à l'intérieur de la pellete représentant 5 à 25 % du volume des pelletes y compris les trous, la disposition des trous à travers une section transversale de la pellete facilitant l'accès des gaz à la substance de la pellete.

2. Catalyseur suivant la revendication 1, caractérisé en ce que le support est composé d'un agrégat de particules d'alpha-alumine qui sont fusionnées ou cimentées ensemble.

3. Catalyseur suivant l'une quelconque des revendications précédentes, caractérisé en ce qu'il présente une aire superficielle spécifique de l'ordre de 0,05 à 10 $m^2/g$ et une absorption d'eau d'au moins 20 %; qu'il comprend 3 à 30 % en pois d'argent et dans lequel au moins 80 % de l'argent est présent sous forme de particules ayant des diamètres équivalents inférieurs à 10.000Å.

4. Catalyseur suivant l'une quelconque des revendications précédentes, caractérisé en ce qu'il contient des cations de métal alcalin et/ou métal alcalino terreux sous forme du nitrate et/ou du nitrite correspondant ou sous une forme capable de réagir pour produire le nitrate ou le nitrite correspondant par réaction avec des substances formant un nitrate et/ou un nitrite en phase gazeuse.

5. Procédé de production d'un oxyde d'alkylène caractérisé en ce qu'il comprend la mise en contact d'une phase gazeuse comprenant une oléfine, de l'oxygène, un agent modifiant la réaction contenant du chlore et une substance formant un nitrate et/ou un nitrite avec un catalyseur suivant l'une quelconque des revendications précédentes.

6. Procédé suivant la revendication 5, caractérisé en ce que le catalyseur comprend un métal alcalin sous une forme qui permet son extraction du catalyseur par l'eau.

7. Procédé suivant la revendication 5 ou la revendication 6, caractérisé en ce que l'oxyde d'alkylène est l'oxyde d'éthylène et que l'oléfine est l'éthylène.

**Patentansprüche**

1. Katalysator für die Herstellung eines Alkylenoxids durch In-Kontakt-Bringen des entsprechenden Olefins und von Sauerstoff mit dem Katalysator, der Silber enthält, das auf einem porösen, hitzebeständigen Träger abgeschieden ist, wobei der Katalysator die Form im wesentlichen zylinderförmiger Pellets hat, bei denen das Verhältnis der Länge zum Durchmesser in dem Bereich von 2:1 bis 1:2 liegt und die mit 3 bis 15 im wesentlichen längslaufenden Löchern durchbohrt sind, die vollständig durch die Pellets hindurchgehen, wobei das Gesamtvolumen der Löcher innerhalb des Pellets 5 bis 25 % des Volumens der Pellets einschließlich der Löcher beträgt, wobei die Anordnung der Löcher über den Querschnitt des Pellets den Zugang von Gas zu der Substanz des Pellets erleichtert.

2. Katalysator nach Anspruch 1, bei dem der Träger aus einem Aggregat von alpha-Aluminiumoxidteilchen besteht, die verschmolzen oder miteinander verklebt sind.

3. Katalysator nach einem der vorhergehenden Ansprüche, der eine spezifische Oberfläche im Bereich von 0,05 bis 10 $m^2/g$ und eine Wasserabsorption von mindestens 20 % hat, der 3 bis 30 Masse% Silber enthält und in dem mindestens 80 % des Silbers in Form von Teilchen mit Äquivalentdurchmessern von weniger als 10.000 Å vorhanden sind.

4. Katalysator nach einem der vorhergehenden Ansprüche, der Alkali- und/oder Erdalkalimetallkationen in Form des entsprechenden Nitrats und/oder Nitrits oder in einer Form enthält, die dazu befähigt ist, durch Reaktion mit nitrat- und/oder nitritbildenden Substanzen in einer Gasphase unter Bildung des entsprechenden Nitrats oder Nitrits zu reagieren.

5. Verfahren zur Herstellung eines Alkylenoxids, bei dem eine Gasphase, die ein Olefin, Sauerstoff, ein chlorhaltiges Mittel zum Modifizieren der Reaktion und eine nitrat- und/oder nitritbildende Substanz enthält, mit einem Katalysator nach einem der vorhergehenden Ansprüche in Kontakt gebracht wird.

6. Verfahren nach Anspruch 5, bei dem der Katalysator ein Alkalimetall in einer Form enthält, die seine Auslaugung aus dem Katalysator durch Wasser erlaubt.

7. Verfahren nach Anspruch 5 oder 6, bei dem das Alkylenoxid Ethylenoxid ist und das Olefin Ethylen ist.